# EUROPÄISCHE PATENTSCHRIFT

(11) **EP 1 833 518 B1**
(45) Veröffentlichungstag und Bekanntmachung des Hinweises auf die Patenterteilung: **08.10.2008**
(21) Anmeldenummer: 05849665.4
(22) Anmeldetag: 30.12.2005
(51) Int. Cl.: A61L 15/42

(54) **VERKÜRZTE WUNDHEILUNGSPROZESSE MITTELS NEUARTIGER FASERVLIESE**
SHORTENED WOUND HEALING PROCESSES BY MEANS OF NOVEL FIBER NON-WOVENS
PROCEDES DE CICATRISATION DE PLAIES RACCOURCIS PAR L'INTERMEDIAIRE DE NON-TISSES D'UN NOUVEAU TYPE

(30) Priorität: 30.12.2004 DE 102004063599; 30.12.2004 US 640896 P
(43) Veröffentlichungstag der Anmeldung: 19.09.2007
(73) Patentinhaber: Bayer Innovation GmbH, 40225 Düsseldorf (DE)
(72) Erfinder: THIERAUF, Axel, 84066 Mallersdorf-pfaffenberg (DE); BAECKER, Iwer, 40213 Düsseldorf (DE); HAISCH, Andreas, 12203 Berlin (DE)
(74) Vertreter: Lütjens, Henning
(86) Internationale Anmeldenummer: PCT/DE2005/002334
(87) Internationale Veröffentlichungsnummer: WO 2006/069567

(56) Entgegenhaltungen:
- EP-A- 0 047 492
- DE-C1- 19 609 551
- US-A- 4 181 127
- US-A- 4 657 006
- US-A- 4 730 611

## Beschreibung

Die vorliegende Patentanmeldung betrifft einen Multilagen-Verband auf der Basis eines Spinn- oder Faservlieses.

Aus dem Stand der Technik ist der feuchte Wundverschluss bekannt (siehe z.B. Blank, Ingo, Wundversorgung und Verbandwechsel, Kohlhammer-Verlag, Stuttgart, 2001, 142, ISBN 3-17-016219-5; Stalick L., Managing and caring for a patient with a complicated wound. Br J Nurs. 2004 Oct 14-27;13(18):1107-9.; Metzger S., Clinical and financial advantages of moist wound management. Home Health Nurse. 2004 Sep; 22(9):586-90).

US 4,730,611 offenbart (vgl. Ansprüche 1 und 4) einen Wundverband umfassend ein Polyurethanschaum-Kissen mit einer hydrophilen, wundzugewandten Seite und einer hydrophoben, wundabgewandten Seite, eine poröse Vliesschicht mit einer Klebeschicht und eine nicht-adhäsive Deckschicht auf der Klebeschicht.

EP 0047492 offenbart (vgl. Seite 2, Absatz 1-3; Ansprüche 1,4,6) einen antiadhäsiven, antimikrobiellen Wundverband umfassend einen antiadhäsiv ausgerüsteten Vlies, welcher mit PVP-Iod-Salbe beschichtet ist.

Problem bei dieser Art der Wundbehandlung ist, dass das Kontaktmedium mit der Wunde, z.B. Verbandmull, Pflaster etc., während des Abheilens mit der Wunde verwachsen kann. Wenn das Kontaktmedium anschließend wieder entfernt werden soll, reißt die Wunde häufig wieder auf, wodurch gerade neu gebildetes Gewebe wieder zerstört und entfernt wird. Es liegt auf der Hand, dass die Wundheilung hierdurch unnötig verzögert wird. Werden Wundauflagen verwendet, die nicht mit der Wunde verkleben, wodurch ein Verwachsen der Wundauflage mit der Wunde verhindert wird, fehlt dem Wunddefekt eine Stütz- und Leitstruktur, an der sich das neu gebildete Gewebe orientieren und wachsen kann. Dieser Umstand führt dazu, dass insbesondere bei tieferen Wunden ein Substanzdefekt entsteht. Außerdem kommt es zu unnötiger und unerwünschter Narbenbildung. Dieses Problem betrifft in der klinischen Praxis alle Wunden, die nicht nur die Epidermis, sondern auch das Korium und ggf. die Subcutis betreffen (sog. "tiefe" Wunden) und eine Rekonstitution nicht nur der epidermalen Schichten, sondern auch des Coriums und ggf. der Subcutis erfordern.

Die Dicke der Epidermis (Oberhaut) ist üblicherweise variabel und kann, je nach Ort, 0,03 bis 4 mm dick sein. Auch das Alter und das Geschlecht haben Einfluss auf die Dicke der Oberhaut. Die Oberhaut besitzt keine Blutgefäße. Sie wird aus Keratinozyten gebildet. Keratinozyten sind Hornzellen, die einen Zellkern besitzen und den Hornstoff, das Keratin, produzieren. Das Keratin ist Wasser abweisend und verleiht der Haut Festigkeit.

Die darunter liegende Lederhaut (Corium) ist eine elastische Hautschicht, die einen hohen Anteil locker verwobenen Bindegewebes enthält. Je nach Ort kann auch sie unterschiedlich dick sein. Am Penis und an den Augenlidern ist sie nur 0,3 mm dünn, während Hand- und Fußsohlen eine Lederhaut von bis zu 2,4 mm Dicke aufweisen.

Die oben genannte Problematik betrifft nicht nur verzögert oder gar nicht heilende Wunden wie das chronische diabetisch-neuropathische Ulcus, Ulcus cruris, Dekubituswunden und sekundär heilende infizierte Wunden, sondern auch reizlose, primär heilende Wunden (z.B. ablative Riß- oder Schürfwunden (bei denen Gewebe abgeschürft und dadurch aus der Wunde entfernt wurde) sowie Spalthautentnahmestellen).

Ausgehend von dem zuvor beschriebenem Stand der Technik haben sich die Erfinder die Aufgabe gestellt, ein Kontaktmedium im weitesten Sinne bereitzustellen (z.B. Verband, Mull, Pflaster), das mit der Wunde in Berührung gebracht werden kann, ohne dass die oben erwähnten Nachteile (Verwachsung des Kontaktmediums mit der Wunde; Zerstörung jüngst gebildeten Gewebes, unnötige Verzögerung der Wundheilung, übermäßige Narbenbildung, Defekt-(heilung)bildung) in Kauf genommen werden müssen. Es war daher ein Ziel der Erfinder, eine Struktur zu entwickeln, mit der es im feuchten Wundverschluss möglich ist, die Struktur im Gewebe zu belassen und auch nach dem Abheilen der Wunde nicht mehr zu entfernen, um somit den Heilungsprozess nicht zu stören, dem sich neu bildenden Gewebe eine Leitstruktur zu geben und um letztendlich eine Narbenbildung zu vermeiden.

Aus der DE-C 196 09 551 sind biologisch degradierbare und/oder resorbierbare Faserstrukturen (Kieselgel-Fasern bzw. -Faserstrukturen) bekannt. Diese lassen sich erhalten, indem aus einer Spinnmasse Fäden gezogen werden, die ggf. getrocknet werden. Die Spinnmasse enthält eine oder mehrere partiell oder vollständig hydrolytisch kondensierte Verbindungen des Siliciums, die sich durch hydrolytische Kondensation ableiten von Monomeren der allgemeinen Formel SiX₄, in der die Reste X gleich oder verschieden sind und Hydroxy, Wasserstoff, Halogen, Amino, Alkoxy, Alkyloxy, Alkylcarbonyl oder Alkoxycarbonyl bedeuten oder sich von Alkyl-Resten ableiten und durch Sauerstoff- oder Schwefel-Atome oder durch Amino-Gruppen unterbrochen sein können.

Bekannt sind weiterhin die in WO 01/42428 und EP-A 01 262 542 beschriebenen Verfahren zur Herstellung eines Hautimplantats bzw. von Zellen, Geweben und Organen, die auf die zuvor genannten Faserstrukturen zurückgreifen. Die WO 01/42428 beschreibt ein Verfahren zur Herstellung eines Hautimplantats, wobei Hautzellen auf die Oberfläche einer Nährlösung aufgebracht werden und die Zellen dann wachsen, dadurch gekennzeichnet, dass auf die Nährlösung ein Flächenelement aus den oben genannten biokompatiblen, biologisch degradierbaren und/oder resorbierbaren Fasern aufgelegt wird. Die Fasern des Flächenelements weisen einen Durchmesser von 5 bis 20 µm auf. Die EP-A 01 262 542 beschreibt dagegen ein Verfahren zur *in vitro*-Herstellung von Zellen, Geweben und Organen, wobei die besagte Fasermatrix (siehe DE-C 196 09 551) als Zellstützsubstanz und/oder Leitstruktur für die von den Zellen gebildete extrazelluläre Matrix dient bzw. den Zellen die Möglichkeit gibt, eine räumliche Anordnung zu finden, die es den Zellen erlaubt, sich zu vermehren und/oder ihre genetisch determinierte Differenzierung zu erreichen.

Die von den Erfindern angebotene Lösung zu dem oben geschilderten Problem macht einen Aspekt der vorliegenden Erfindung aus und besteht in der Verwendung der aus der DE-C 196 09 551 bekannten Fasern für die Herstellung eines Multilagen-Verbandes gemäß Patentanspruch 1. Dabei werden die genannten Fasern zu einem Vlies verarbeitet, das dann mit sämtlichen konventionellen Verbandmitteln, insbesondere mit Verbandmitteln, die unmittelbar auf oder in die Wunde eingebracht werden, kombiniert werden kann. Diese Kombination wird hier und nachfolgend als Multilagen-Verband bezeichnet, auch wenn es sich nicht um einen typischen Verband, sondern um ein Pflaster, eine Kompresse, o.ä. handelt. Ein Aspekt der vorliegenden Erfindung betrifft also einen Multilagen-Verband, der wenigstens den folgenden Aufbau hat: ein Vlies 1, das mit der Wunde in Berührung kommen soll, und eine Membran 3, die wasserdicht ist und mindestens ein in Wasser unlösliches Polymer aufweist.

Der oben erwähnte Multilagen-Verband hat den folgenden Aufbau:
ein Vlies 1, das mit der Wunde in Berührung kommen soll, und
eine Membran 3, die wasserdicht ist und mindestens ein in Wasser unlösliches Polymer aufweist, wobei die Membran 3 entweder ein Klebepflaster 3 ist und einen klebefähigen Anteil umfaßt, der mit der die Wunde umgebenden Haut verklebt, oder keinen klebefähigen Anteil umfaßt und mit der die Wunde umgebenden Haut nur verklebt, wenn auf die die Wunde umgebende Haut ein Kleber aufgetragen worden ist,
und wobei zwischen Membran 3 und Vlies 1 eine lockere, leicht zu lösende Haftverbindung oder gar keine Haftverbindung besteht, wobei das Vlies 1 biologisch degradierbare und/oder resorbierbare Faserstrukturen aufweist, die sich erhalten lassen, indem aus einer Spinnmasse Fäden gezogen werden, wobei die Spinnmasse eine oder mehrere partiell oder vollständig hydrolytisch kondensierte Verbindungen des Siliciums enthält, die sich durch hydrolytische Kondensation ableiten von Monomeren der allgemeinen Formel SiX4, in der die Reste X gleich oder verschieden sind und Hydroxy, Wasserstoff, Halogen, Amino, Alkoxy, Alkyloxy, Alkylcarbonyl oder Alkoxycarbonyl bedeuten oder sich von Alkyl-Resten ableiten und durch Sauerstoff- oder Schwefel-Atome oder durch Amino-Gruppen unterbrochen sein können,
und wobei zwischen Membran 3 und Vlies 1 eine lockere, leicht zu lösende Haftverbindung oder gar keine Haftverbindung besteht.

Besonders bevorzugt ist ein Multilagen-Verband, wie im vorstehenden Absatz beschrieben, wobei die Reste X gleich sind und Ethyl bedeuten.

Weiterhin bevorzugt ist ein Multilagen-Verband, wie er im vorletzten Absatz beschrieben wurde, wobei die hydrolytische Kondensation in Gegenwart von einer (oder mehreren) Aminosäuren und/oder von einem (oder mehreren) Peptiden und/oder von einem (oder mehreren) DNA-Molekül(en) bzw. -Fragmenten erfolgt. Dabei sind die Reste X in der Formel SiX₄, des Silans ggf. gleich und bedeuten ggf. Ethyl. Die Gegenwart der Aminosäure(n) und/oder Peptide und/oder DNA/DNA-Fragmente bewirkt deren Einbau in die Faser, sei es mittels kovalenter oder nicht-kovalenter Bindung. Die Aminosäuren/Peptide/DNA/DNA-Fragmente werden nach Einbringen des Multilagen-Verbands in die Wunde, korrespondierend zur Degradation der Faser, aus dieser freigesetzt. Hierbei wird einerseits die Menge an freigesetztem Material (Aminosäuren/Peptide/DNA/DNA-Fragmente) durch die Menge des in die Faser eingebauten Materials (Aminosäuren/Peptide/DNA/DNA-Fragmente), andererseits aber auch die Freisetzungsgeschwindigkeit aus der Faser durch die Degradationsgeschwindigkeit der Faser bestimmt. Durch die nachfolgend beschriebene (Seite 14) Fasereigenschaft (1) Zelladhäsion wird ein Einbau der Aminosäuren/Peptide/DNA bzw. DNA-Fragmente in die proliferierenden Zellen in besonderem Maße ermöglicht, wodurch insbesondere auch ein direkter Einfluß der in der DNA/den DNA-Fragmenten kodierten Information auf die Zellen gewährleistet ist. Dieser Umstand erweist sich bei Wunden mit verminderter regionärer oder gar systemischer Stoffwechsellage als besonders wichtig und hilfreich, da hierdurch eine externe Versorgung der Wundregion mit für den Zellstoffwechsel notwendigen Aminosäuren gewährleistet und hierdurch erstmals eine Wundheilung ermöglicht wird.

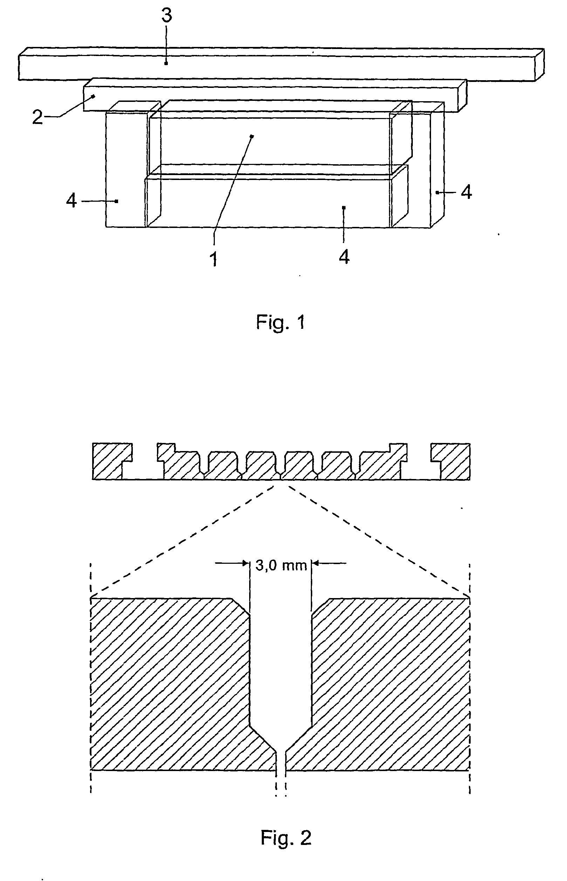

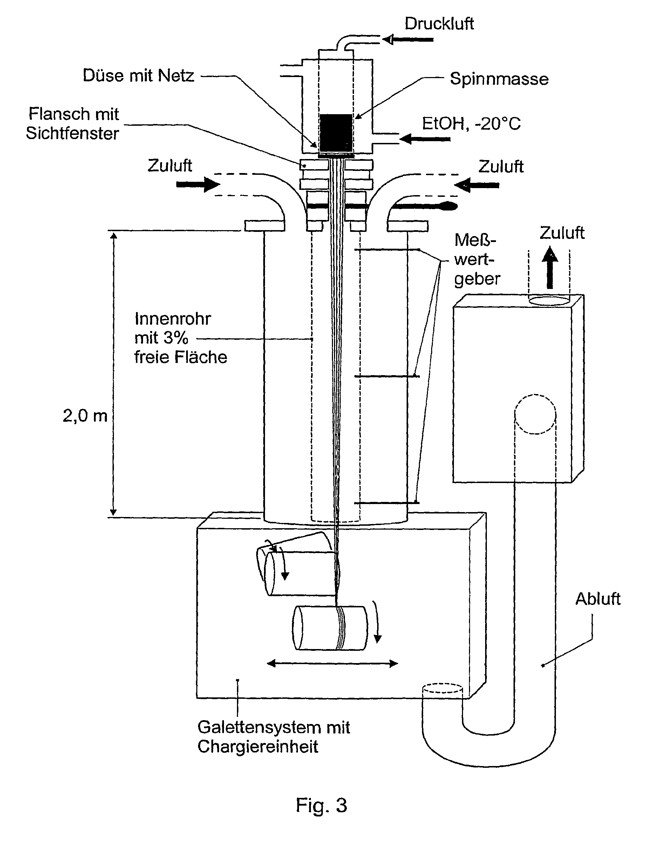

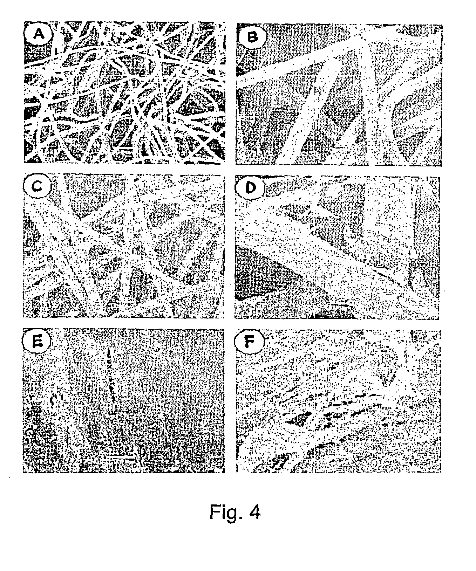

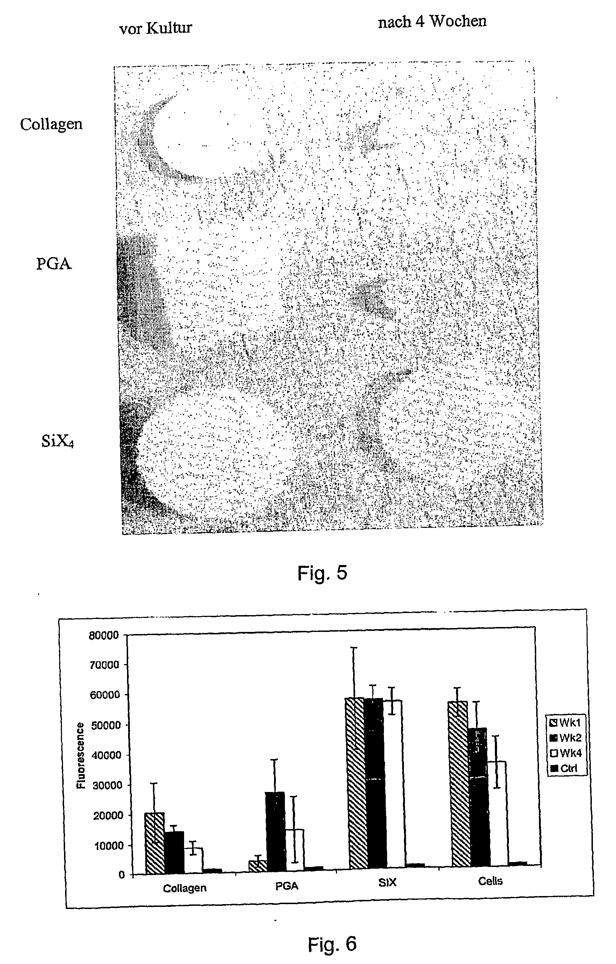

Vorliegend werden die Figuren 1 bis 6 kurz erläutert. Diese Figuren zeigen
- den schematischen Aufbau eines erfindungsgemäßen Multilagen-Verbands (Fig. 1);
- die Skizze einer Lochdüse in einer Düsenplatte (Fig. 2);
- eine schematische Darstellung eines Galetten-Systems und eines Klima-Aggregats (Fig. 3);
- die Anhaftung von Zellen an der Faseroberfläche, dargestellt mittels SEM (Fig. 4), wobei die Fig. 4A und B die erfindungsgemäß verwendete SiX₄-Faser ohne Zellen, die Fig. 4C und D die SiX₄-Faser mit Zellen sowie deren ausgezeichnete Adhäsion und Verteilung und die Fig. 4E und F eine Kollagen-Faser mit Zellen zeigen, deren Morphologie in Folge der rauen Natur der Kollagenmatrix nur schwierig zu ermitteln ist;
- die Form- und Schrumpfungsbeständigkeit der erfindungsgemäß verwendeten SiX₄-Faser im Vergleich zu Kollagen- und PGA-Fasern, wobei die linke Spalte, von oben nach unten, eine Kollagen-, eine PGA- und eine erfindungsgemäße SiX₄-Faser vor Beginn der Zellkultivierung zeigt und wobei die rechte Spalte, von oben nach unten, eine Kollagen-, eine PGA- und eine erfindungsgemäße SiX₄-Faser vier Wochen nach Beginn der Zellkultivierung zeigt (Fig. 5); und
- die Stoffwechselaktivität von Zellen (dermalen Fibroblasten), gemessen als Fluoreszenz mittels des Alamar-Blau-Assays, nach 1, 2 und 4 Wochen ihrer Kultivierung mit einer Kollagen-, PGA-, der erfindungsgemäßen SiX₄ (SIX)-Faser und ohne Faser im Vergleich (Fig. 6).
Wie bereits erwähnt, lassen sich aus den in der DE-C 196 09 551 beschriebenen Kieselgel-Fasern bzw. -Faserstrukturen (der chemischen Formel, bezogen auf die Monomer-Einheit, SiO₂. ₓOHₓ bzw., bezogen auf das Polymer, Siₙ(OH)₂ₓO₂ₙ₋ₓ, mit x = 0-1), die erfindungsgemäß als Stütz- und Leitstruktur in die Wunde eingebracht und somit in direkten Kontakt mit dem Gewebe gebracht werden, feuchte Vliese herstellen. Bei den Vliesen werden Faservliese und Spinnvliese unterschieden. Insbesondere die Spinnvliese, die aus endlosen Einzelfasern bzw. - filamenten hergestellt werden, sind für 3D-Anwendungen vorteilhaft, während die Faservliese für 2D-Anwendungen besonders geeignet sind.

Das Verfahren zur Herstellung der Einzelfasern/-filamente kann der Patentschrift DE-C 196 09 551 entnommen werden. Dabei wird der Fachmann berücksichtigen und wissen, dass zahlreiche Parameter wie Temperatur, Druck, molares Verhältnis der Einzelkomponenten, chemische Beschaffenheit der Edukte, des Lösungsmittels oder des Katalysators variiert werden können, um ggf. besonders geeignete Fasern und Vliese (mit hoher oder niedrigerer Biodegradier- und Bioresorbierbarkeit) zu erzeugen.

Das in der DE-C 196 09 551 beschriebene Verfahren soll hier noch einmal, ggf. etwas ausführlicher, dargestellt werden. Erfindungsgemäß bevorzugt ist die Verwendung von TEOS (Tetraethoxysilan) als Silan in einem Sol-Gel-Prozeß, obwohl alle in der DE-C 196 09 551 genannten Silane, oder Gemische von mindestens zwei von ihnen, gleichfalls verwendet werden können. In Anwesenheit von wässrig-alkoholischer Lösung (erfindungsgemäß vorzugsweise ein Ethanol/Wasser-Gemisch), das einerseits (Ethanol oder Ethanol/Wasser) als Lösungsmittel, andererseits (Wasser) aber auch als Reaktionspartner für die hydrolytische Kondensation dient, wird bei Raumtemperatur oder auch leicht reduzierter Temperatur (12-15°C) ein Kondensationsprodukt eines geeigneten Kondensationgrades hergestellt. Ein bevorzugter Katalysator für die Kondensation ist eine organische Säure wie Zitronen-, Bernstein- oder Weinsäure. Diese Säuren stellen den pH des Reaktionsgemisches auf etwa 3-4 ein. Der pH muß auf jeden Fall bei 7 oder darunter liegen, da sich im Alkalischen Teilchen bilden oder der Reaktionsansatz geliert.

Ggf. wird das Produkt der Kondensation durch Filtration und Entzug von Lösungsmittel auf eine geeignete Viskosität gebracht, wie es auch in der DE-C 196 09 551 beschrieben ist. Es läßt sich bei Temperaturen unter 0°C eine gewisse Zeit (mehrere Stunden bis wenige Monate) unter Erhalt eines sog. Spinnsols (einer Spinnmasse), verwahren, da die Kondensation bei Temperaturen unterhalb 0°C nur sehr langsam abläuft. Vorzugsweise weist das Spinnsol einen Feststoffgehalt (Feststoff bedeutet (Teil-)Kondensationsprodukte, also Oligomere bzw. oligomere Strukturen) von ca. 10 % auf (d.h., der Anteil an Lösungsmittel beträgt ca. 90 %). Ebenfalls bevorzugt ist erfindungsgemäß ein Zeitraum von 2-3 Tagen vom Beginn der Kondensationsreaktion bis zum Erhalt des Spinnsols.

Das Spinnsol wird schließlich in einen vorgekühlten (<0°C) Druckbehälter gegeben, aus dem es in Form langer, nicht-reißender bzw. reißfester Fäden durch kleine Düsen unter Druck herausgepresst wird. Die Fäden haben, je nach Größe der Düsen, einen Durchmesser von etwa 10 bis 100 µm und eine Länge von mehreren (z.B. 3-5) m. Wenn die Fäden aufgewickelt werden, können sie ggf. durch Ziehen (Recken) während des Aufwickelns (ggf. in wässrig-alkoholischer Atmosphäre) weiter verlängert und ihr Durchmesser weiter reduziert werden. Das Aufwickeln der Fäden erfolgt mit Spinngeschwindigkeiten von 100-1000 m/min, vorzugsweise mit Geschwindigkeiten von etwa 200 m/min. Die so gesponnenen Fäden lassen sich über eine Rolle auf ein Trägerband verwirbeln. Die Fäden auf dem Trägerband können nun verschiedenen Temperaturen ausgesetzt werden, indem das Trägerband mit einer Laufgeschwindigkeit von 1-10 cm/min durch verschiedene Temperaturzonen wandert, so dass über eine weiter ablaufende Kondensationsreaktion die Zahl der in der Faser verbleibenden OH-Gruppen (also die Biodegradier- und Bioresorbierbarkeit der Faser) nach Wunsch eingestellt werden kann (bevorzugt ist es erfindungsgemäß, die Fäden in Form von Gelfäden auf dem Trägerband schlagartig auf -35°C abzukühlen).

Die verwirbelten Fäden (Endlosfasern) werden anschließend zu (Spinn- oder Faser-)Vliesen verpresst. Die Verpressung erfolgt über eine Andruckrolle. Hierbei werden häufig auch Nadelstühle (Andruckrollen mit Nadeln) eingesetzt. Durch die Auf- und Abbewegung der Nadeln entsteht ein Walkprozess, der dem Vlies eine zusätzliche Festigkeit verleiht. Der Anpressdruck der Rolle mit und ohne Nadeln kann frei eingestellt werden. Der erfindungsgemäß bevorzugte Pressdruck liegt typischischerweise bei 1-10 MPa. Es folgt eine Temperaturbehandlung des Vlieses, wobei sich die Temperaturen in dem Bereich von -35°C bis +65°C bewegen. Bevorzugt ist eine Temperatur von unter -5°C, besonders bevorzugt eine Temperatur von unter -20°C. Durch diese Temperaturbehandlung erhält man ein strukturell festes Gelege mit einer gleichzeitig ausreichenden Anzahl von Silanol-, also nicht-kondensierten OH-Gruppen, im Vlies. Die Anzahl der nicht-kondensierten OH-Gruppen steuert die (Bio)Resorbierbarkeit: je mehr nicht-kondensierte OH-Gruppen vorliegen, umso größer ist die (Bio)Resorbierbarkeit. Durch die Variation der Haltezeiten bei unterschiedlichen Temperaturen kann die Anzahl der OH-Gruppen gezielt eingestellt werden. Vorzugsweise weisen die Fasern des erfindungsgemäß zu verwendenden Vlieses etwa eine OH-Gruppe pro 5-10 Si-Atome auf, was in obiger Formel für die Monomer-Einheit SiO₂₋ₓOHₓ mit x = 0,1-0,2 bedeutet.

Ggf. wird das Vlies im Anschluß auf eine Temperatur von 50°C oder auf eine Temperatur oberhalb von 50°C gebracht, um evtl. noch vorhandenes Wasser und Ethanol (z.B. aus dem Lösungsmittel, aber auch aus den Resten des Ausgangssilan, insbesondere bei TEOS als Ausgangssilan) bis zu dem gewünschten Ausmaß, meist aber nicht völlig, zu entfernen. Denn es ist erfindungsgemäß wünschenswert, dass die (z.B. Spinn-)Vliese eine ausreichende Restfeuchtigkeit erhalten, um den thermodynamisch ungünstigen Gelzustand (Faser mit nicht-kondensierten OH-Gruppen im Vlies) gegenüber dem bei Raumtemperatur günstigeren SiO₂ (Faser ohne nicht-kondensierte OH-Gruppen, also Glasfaser, im Vlies) zu stabilisieren. Derart hergestellte Spinnvliese können in geschlossenen (luftdichten) Verpackungen ihren Gelzustand über mehrere Monate erhalten. Insbesondere die Anwesenheit von Restethanol, aber auch die von Wasser, erweist sich hierbei als vorteilhaft. Dies beruht wohl darauf, dass in einer (gesättigten) Ethanol-Atmosphäre die Kondensation in Richtung SiO₂ nicht weiterläuft. Im Gegenteil, sie kann sogar teilweise umgekehrt werden, was letztendlich die Bioresorbierbarkeit der Faser ausmacht.

Das beschriebene Verfahren wird zur Herstellung von Spinnvliesen verwendet. Aber auch Faservliese - oft Nadelvliese genannt - lassen sich so herstellen. Dabei werden die Fasern nach dem Spinnvorgang in Stücke geschnitten. Diese Stapelfasern haben Längen von 0,1-10 cm. Die Stapelfasern werden anschließend auf ein Trägerband abgeworfen, angepresst, genadelt und einer Temperaturbehandlung wie oben beschrieben unterworfen. Im Gegensatz zu den Spinnvliesen haben Faservliese keine ausgeprägte 3D-Struktur. Sie werden daher häufig in 2D-Anwendungen (oberflächige Verletzungen im oberen epidermalen Niveau) eingesetzt. Erfindungsgemäß ist der Einsatz von Faservliesen daher insbesondere bei der Versorgung von oberflächigen Wunden (oberes epidermales Niveau) vorteilhaft. Allgemein läßt sich sagen, Faservliese haben generell höhere Festigkeiten in der Faserebene und sind daher für mechanisch belastete Stellen, z.B. Haut über Muskeln besser geeignet.

Die oben beschriebene Herstellung der Faser erfolgt im Sol-Gel-Verfahren auf einer Maschine mit z.B. einer Länge von ca. 5 m, einer Breite von ca. 2 m und einer Höhe von ca. 6 m. Das Gewicht der Maschine führt im Bereich unter dem Spinnturm zu einem Druck von 850 bis 1.000 kg/m². Die Maße der Maschine können aber je nach Ausstattung und Leistungsfähigkeit auch erheblich von den hier genannten Größen abweichen. Zur Produktion benötigt die Maschine einen Kühlwasserkreislauf mit ausreichender Wasserversorgung sowie vorzugsweise einen Starkstrom-Anschluß.

Zum gegenwärtigen Zeitpunkt erfolgt die Herstellung der Faser erfindungsgemäß auf einer Klima-Spinnanlage. Die Klima-Spinnanlage wird von einem umluftbetriebenen Klima-Aggregat mit Zuluft versorgt, die eine definierte Temperatur und Feuchte hat. Bevorzugt sind Temperaturen im Bereich von 10 bis 40°C, insbesondere Temperaturen von etwa 20°C. Das Aggregat ist ein Klima-Prüfschrank der Firma Weiß Umwelttechnik GmbH mit der Bezeichnung SB22/160/40-UKA. Es wurde für den Umluftbetrieb von der Firma Weiß nachgerüstet. In einen 2 m langen, isolierten Spinnturm mit dem Außendurchmesser von 680 mm ist zur Vermeidung von störender Konvektion durch den Umluftbetrieb ein Innenrohr eines Durchmessers von 300 mm mit 3 mm Rundlochbohrungen eingesetzt. Der Spinnturm ist an eine Box angeschlossen, in der sich die Wickelvorrichtungen für die oben erwähnten Endlosfasern bzw. -filamente befinden. Die Scheiben der Box wurden für eine ausreichende Isolierung aus Fensterglas (Dicke 24 mm) gefertigt und haben einen K-Wert von 1.1 Die Abluft aus der Box wird dem Klimaaggregat wieder zugeführt und dort aufbereitet. Der Meßwertgeber für das Klima ist hierbei nicht der Innenraum des Aggregates, sondern ein externer Fühler in dem Spinnschacht. Zur Ansteuerung des Klimageräts, die auch manuell möglich ist, wurde ein PC angeschlossen, der mit der entsprechenden Software des Herstellers versehen war. Mit Hilfe des Programms PCC_WIN, Version 1.05, können Temperatur- und Feuchte-Programme sowie alle anderen nötigen Einstellungen für das Aggregat vorgegeben werden. Bei laufendem Spinnvorgang kann ein Plotter die Temperatur und Feuchte im Spinnschacht zeitabhängig darstellen. Ein zusätzlicher Temperaturfühler wurde zur Messung der Außentemperatur angeschlossen, auch dieser Wert wird digital erfasst. Für eine Aufrüstung der Spinnanlage mit einer Prozessleit-Technik sind alle wesentlichen Messstellen mit einem Analogausgang ausgestattet.

Die Zu- und Abluftverbindungen zwischen Klima-Aggregat und Spinnturm bzw. Box bestehen aus flexiblen, doppelt isolierten Schläuchen mit einem Innendurchmesser von 100 mm (Außendurchmesser: 250 mm). Die Anschlüsse wurden jeweils mit Armaflex verkleidet. Da beim Spinnvorgang ggf. Ethanol aus der Spinnmasse freigesetzt wird, welches sich in dem geschlossenen Kreislauf von Klima-Aggregat, Spinnturm und Box akkumulieren kann, wurde die Anlage mit einer Gaswarneinrichtung der Firma GfG Gesellschaft für Gerätebau ausgerüstet. Je ein auf Ethanol geeichter Messwertgeber mit der Bezeichnung MWG 0238 EX wurde in die Box in unmittelbarer Nähe der Motoren bzw. in den Prüfraum des Klima-Umluftaggregates montiert. Eine Auswerteeinheit (GMA· 100-BG) gibt einen ersten Alarm, wenn die Konzentration des Ethanol in der Raumluft 25 % der unteren Explosionsgrenze des Ethanol erreicht hat, sowie einen zweiten Alarm bei Erreichen von 50 % der unteren Explosionsgrenze. Ebenso wird bei Ausfall oder Fehlfunktion der Messwertgeber ein Alarm ausgelöst.

Am oberen Ende des Spinnturms ist ein Balgzugschieber und ein Übergangsflansch mit drei Anschlussmöglichkeiten angebracht, auf den der doppelwandige und nach außen isolierte Spinnkopf montiert werden kann. Nach dem Prüfbericht der Druckprüfung ist der Spinnkopf für einen Druck bis 50 bar (5 x 10⁶ Pa) geeignet. Mit einem Innendurchmesser von 45 mm fasst der Spinnkopf 0.33 Liter Spinnmasse.

Die Düsenplatte wird von unten an den Spinnkopf angebracht. Die Platte mit einem Durchmesser von 89 mm hat einen 1.5 mm tiefen Einsatz, in den ein in Aluminium gefasstes Edelstahlgewebe eingesetzt wird. Das Drahtgewebe ist zweilagig, wobei die erste Lage eine Maschenweite von 80 µm besitzt.

Die zweite Stützlage hat eine Maschenweite von 315 µm. Die Aluminiumfassung des Drahtgewebes ist so konstruiert, dass sie, wenn das Netz in die Düsenplatte gelegt wird, um 0.5 mm übersteht. Wird die Platte mit dem Netz mit 15 Nm an den Spinnkopf geschraubt, so sorgt der verpresste Al-Ring für die nötige Abdichtung zwischen Spinnkopf und Platte. Verwendet wurden 7- bzw. 19-Loch-Düsenplatten. Die Vorbohrung eines Lochs ist 3.0 mm breit, der Lochdurchmesser beträgt 0.15 mm. Bei einer Kapillarlänge von 0.45 mm ergibt sich ein L/D Verhältnis von 3. Eine Skizze einer Lochdüse in einer Düsenplatte zeigt Fig. 2.

Der doppelwandige Spinnkopf wird mit Hilfe eines Thermostaten der Firma LAUDA (Bezeichnung RE 112) temperiert, wobei der Zu- und Ablaufschlauch zur Isolierung mit Armaflex verkleidet sind.

In die drei Anschlüsse des Übergangsflansches zwischen Spinnkopf und Turm sind Schaugläser so eingesetzt, dass beim Spinnvorgang der Fadenaustritt aus den Düsen beobachtet werden kann. Bei der Konstruktion einer Vorrichtung zum Ablegen der Filamente wurde neben einer Wickeleinheit auch die Möglichkeit einer Faserablage über eine Gasförderdüse berücksichtigt. Hierzu wurde für den Verzug der Filamente ein System aus 2 Galetten einer Länge von 159 mm und 220 mm konstruiert, wobei die hintere Galette gegenüber der vorderen um 8° geneigt ist. Der Antrieb erfolgt über eine Motor-Tacho-Kombination (Bez. S4.3 G 60) und Getriebe (Bez. 381, 3.71: 1) der Firma Faulhaber. Die Rotationsfrequenz der ersten Galette wird automatisch von der zweiten Galette übernommen.

Über einen Verzugsregler kann die zweite. Galette um bis zu 10 % schneller rotiert werden. Eine dritte Galette dient als Wickler und kann unabhängig von der Verzugseinheit betrieben werden. Sie besteht aus einem Dorn, der ebenfalls über eine Motor-Tacho-Kombination der Firma Faulhaber angetrieben wird, auf den Dorn kann eine Papprolle gespannt werden. Diese Papprolle ist aus fünf einzelnen Kreissegmenten zusammengesetzt, die mit einer Federkonstruktion zu einem Kreisdurchmesser von 159 mm verbunden sind. Im entspannten Zustand verringert sich der Durchmesser der Rolle von 159 mm auf 143 mm. Die fünf Segmente sind außen mit einer Teflonfolie beklebt.

Die dritte Galette ist auf eine Chargiereinheit der Firma Isel-Automation montiert. Mit einem Schrittmotor der Bezeichnung 160 MCM kann die Wickelvorrichtung auf einer Länge von 500 mm chargiert werden. Die Chargierfrequenz aus dem Hin- und Rückweg der Einheit kann auf Werte von 2 bis 16 min⁻¹ eingestellt werden, mit einer zweiten Steuereinheit kann die Galette manuell verfahren werden. Eine Steuerungseinheit für die Galetten-Motoren und den Chargier-Schrittmotor wurde aus Controllern (1T 142-C), einer 1-Achs-Schrittmotorsteuerung mit Adapterkarte und einer Steuerkarte (UMS 6) der Firma Isel-Automation gefertigt. Eine schematische Darstellung des Galetten-Systems und des Klima-Aggregates ist in Fig. 3 zu finden.

Es ist erfindungsgemäß vorteilhaft, wenn die Vliese (im weiteren Text wird nur noch der Begriff Vlies synonym für Spinnvlies und Faservlies verwendet) in oder auf Wunden gelegt werden, die entweder primär serös sezerniert oder sekundär mit extern zugeführten physiologischen Lösungen wie physiologischer Kochsalz-Lösung (0,9%) versehen werden. Durch die Resorption des Vlieses in der Wunde während des Heilungsvorganges entfällt die Notwendigkeit, das Vlies während oder nach der Wundheilung entfernen zu müssen. Die Faserdichte ist durch den Verdichtungsvorgang im Vlies frei einstellbar und wird entsprechend der Art und Tiefe der Wunde variiert. Typische Anwendungsbereiche liegen bei Wundtiefen von 1 - 20 mm, vorzugsweise 2-12 mm, im wesentlichen abhängig von der Dicke der Epidermis.

Darüber hinaus kann durch Variation der Herstellungsparameter der Endlosfasern und der Vliese (Wahl der Gruppen X in SiX₄, Wahl der Reaktionsbedingungen für die hydrolytische Kondensation, somit Wahl des Anteils nicht-polymerisierter OH-Gruppen, etc., siehe DE-C 196 09 551) die Resorptionszeit der Faser und damit des Vlieses eingestellt und an die Wundverhältnisse angepasst werden. So kann je nach Anforderung eine Resorptionszeit von 3 bis 180 Tagen eingestellt werden, wobei das Intervall nach oben beliebig stufenlos verlängert werden kann. Dabei haben die Erfinder festgestellt, dass durch Variation der Anzahl der OH-Gruppen in der Faser bzw. dem Vlies sowie durch Zugabe von morphogenen Faktoren (Heilungsbeschleunigern), die chemisch über OH-Gruppen oder physikalisch über Physisorption auf der extrem großen, hoch hydrophilen Oberfläche an die Faser (im Vlies) gebunden werden, die Resorptionszeit des Vlieses eingestellt werden und an die feuchten Wundverhältnisse angepasst werden kann. Es hat sich als vorteilhaft herausgestellt, wenn an jedem fünften bis zehnten Si-Atom eine funktionale OH-Gruppe vorhanden ist. Unter einer funktionalen OH-Gruppe wird hier eine mögliche freie Reaktionsstelle in Form einer OH-Gruppe verstanden, wo z.B. Medikamente wie Antibiotika, Antimykotika, Steroide und allgemein Medikamente mit lokaler oder systemischer Wirkung über Wasserstoffbrücken oder Kondensation an das Vlies gekoppelt werden können, die dann später in der Wunde Zug um Zug freigesetzt werden (Drug Release). Die Resorptionszeiten bewegen sich dann bei ca. 30 Tagen, und die Abbauprodukte (SiO₂ bzw. SiO(OH) als Nanopartikel) haben typischerweise Durchmesser von 0,5-1 nm. Eine genaue Strukturaufklärung erfolgt dabei über Si-Festkörper-NMR, insbesondere durch die Messung der Q4-Mode. Erfindungsgemäß bevorzugte Drücke liegen typischerweise bei 1-10 bar (10⁵-10⁶Pa), vorzugsweise bei 2-3 bar, die Reaktionszeiten im Spinnprozess betragen vorzugsweise 20-60 s, während die Temperaturen (im Spinnprozess) vorzugsweise bei 15-23°C, insbesondere bei etwa 20°C gehalten werden.

Aufgrund der Fasergeometrie wird sogar eine Wundheilungsbeschleunigung (Tissue Guiding) beobachtet. Hierbei dienen die zwei- bzw. dreidimensional angeordneten, hoch hydrophilen Gelfasern als physikalische Leitstruktur, an der die proliferierenden Zellen anhaften und eine lokalisationsgerechte, zumeist kollagene Matrix ausbilden können. Da die chemische Umgebung der Faser annähernd pH-neutral (pH 7,0 ± 0,2) ist und keine organischen Zerfallsprodukte entstehen, treten keinerlei Fremdkörper-Reaktionen bzw. Irritationen der sich neu ausbildenden Zellen auf. Vielmehr wird durch eine Akkumulation der oben genannten morphogenen Faktoren (Steroide, Cytokine, TNF-alpha, TGF-beta 1/2, Interleukine wie IL-1, PDGF, EGF etc.), die von der Wunde sezerniert werden, die Wundheilung kontinuierlich physiologisch stimuliert. Im Gegensatz zu gelartigen und viskosen Matrix-Materialien aus dem organischen Bereich (Kollagen, Hyaluronsäure, Fibrin) besteht bei der anorganischen Faser keine potentielle Infektionsgefahr mit bekannten (HIV, HBV, BSE, Prionen etc.) und bis dato nicht bekannten Infektionsquellen. Darüber hinaus sind bei anorganischen Materialien die Materialparameter äußerst genau definierbar und einstellbar. Hierdurch werden im Vergleich zu organischen Materialien die Qualität und das Eigenschaftsprofil erheblich verbessert.

Die erfindungsgemäß verwendete Faser unterscheidet sich von herkömmlichen biodegradierbaren bzw. bioresorbierbaren Biomaterialien durch wenigstens die folgenden vier Merkmale bzw. Eigenschaften deutlich: sie erlaubt (1) eine verbesserte Zelladhäsion (Anhaftung der Zellen an die Faser), und sie ermöglicht (im völligen Gegensatz zu bekannten Materialien) (2) eine Zellproliferation (Zellvermehrung), (3) eine Aufrechterhaltung von Form und Stabilität der Faser sowie (4) eine langfristige Aufrechterhaltung der Zellproliferation und des Zellstoffwechsels.

### (1) Zelladhäsion:

Die besondere Fasergeometrie und -morphologie erlaubt gegenüber herkömmlichen bioresorbierbaren Materialien (wie Polyglykolsäure (PGA), Alginaten und Kollagen) eine ausnahmslos schnellere Initialisierung und qualitativ bessere Anhaftung von Zellen an der Faseroberfläche (diese Verbesserung wird in Fig. 4 dargestellt). Hierdurch wird eine schnelle und sichere Verteilung/Aussprossung von Zellen entlang der in der Wunde befindlichen Faser in alle Bereiche der Wunde garantiert. Ausgehend von an Fasern anhaftenden Zellen wird darüber hinaus eine zuverlässige Verteilung von neu gebildeten Zellen auch entfernt zur Faser begünstigt (Stichwort: cell compound proliferation). Diese vorteilhafte Eigenschaft der erfindungsgemäß verwendeten Faser konnte unter Verwendung von Scanning-Elektronen-Mikroskopie (SEM), histologischen und immunhistologischen Untersuchungen sowie Konfokaler Mikroskopie eindrucksvoll dargestellt werden.

### (2) Zellproliferation:

Die besondere Fasergeometrie und -morphologie erlaubt gegenüber herkömmlichen biodegradierbaren/bioresorbierbaren Materialien einen schnelleren/früheren Beginn, eine Beschleunigung/Erhöhung sowie eine längere Dauer/Aufrechterhaltung der Zellproliferation. Diese Eigenschaft begünstigt die unter (1) aufgeführten Vorteile bzgl. der Nutzung der Eigenschaften der Faser, den Zellen eine schnellere und qualitativ bessere Anhaftung von Zellen an der Faser-Oberfläche zu ermöglichen.
Die Stoffwechselaktivität der Zellen, gemessen mittels des Alamar-Blau-Assays als Referenzparameter für die Zellproliferation und Aktivität der Zellen, ist nach einem kurz- bis mittelfristigen Zeitraum von 1, 2 und 4 Wochen im Vergleich zu herkömmlichen Materialien wie PGA und Kollagen deutlich gesteigert. Das Verhältnis der Stoffwechselaktivität der Zellen mit der Matrix PGA bzw. Kollagen bzw. SiX₄ (erfindungsgemäße Faser) ist 1 : 5 : 11 (1 Woche), 2,5 : 1 : 6 (2 Wochen) und 1,2 : 0,8 : 6 (4 Wochen). Anfänglich (nach 24 h) betrug dieses Verhältnis allerdings nur 1 : 4,5 : 4. Dies belegt, daß die erfindungsgemäß verwendete Faser ihre Vorteile erst nach einem längeren Zeitraum von wenigstens einer Woche, besser 4 Wochen entfaltet.

### (3) Aufrechterhaltung von Form und Stabilität der Faser:

Wie unter Verwendung von SEM, histologischen und makroskopischen Untersuchungen bestätigt werden konnte, erlaubt die erfindungsgemäße Faser gegenüber herkömmlichen bioresorbierbaren Materialien eine lang anhaltende Aufrechterhaltung der dreidimensionalen Formgebung und eine verzögerte Kontraktion der dreidimensionalen Faseranordnung (Fasergeometrie und -morphologie bleiben weitestgehend erhalten): Herkömmliche Materialien wie PGA und Kollagen schrumpfen (sintern) während eines Zeitraums von 4 Wochen um den Faktor 4 bzw. 6 und verlieren darüber hinaus ggf. auch ihre Form, während die erfindungsgemäße Faser über diesen Zeitraum Form und Stabilität vollständig aufrecht erhalten kann (dieses Phänomen wird in Fig. 5 dargestellt). Dieser Umstand gewährleistet einen sicheren Aufbau von neu gebildetem Gewebe und garantiert auch bei großen Wunden eine ausreichende Diffusion von Nährstoffen und Stoffwechselprodukten. Darüber hinaus wird, anders als mit den weniger formstabilen Materialien, die aus dem Stand der Technik für diesen Zweck bekannt sind, eine Neubildung von Gefäßen ermöglicht und gefördert. Damit ist mit dem erfindungsgemäßen Material, und anders als mit den aus dem Stand der Technik bekannten Materialien wie PGA oder Kollagen, eine Gefäß- und Gewebeneubildung auch bei großen Wunden und somit deren Heilung erstmals möglich. Ein wichtiger Aspekt diesbezüglich ist die Formstabilität der erfindungs gemäßen Faser, gerade auch im Hautbereich, die eine mechanische Stabilisierung bewirkt. Das neu gebildete Gewebe kann bei Verwendung des erfindungsgemäßen Multilagen-Verbandes ausreichend mit Nährstoffen versorgt werden. Diese Versorgung erfolgt nicht nur durch Diffusion, sondern auch durch direkten Transport der Nährstoffe durch die neu gebildeten Gefäße/Gewebe im offenporigen Vlies. Was die Formbeständigkeit betrifft, addieren sich die in (1) und (2) beschriebenen positiven Eigenschaften (Zellproliferation, Zelladhäsion).
Dies konnte unter Verwendung von folgender Analytik eindrucksvoll dargestellt werden: Scanning Elektronen Mikroskopie (SEM), Histologie, Mikroskopie

### (4) Langfristige Aufrechterhaltung von Zellproliferation und Zellstoffwechsel:

Die besondere Fasergeometrie und -morphologie erlaubt gegenüber herkömmlichen bioresorbierbaren Biomaterialien eine lang anhaltende Aufrechterhaltung der Zellproliferation, wodurch ein(e) zuverlässige(r) Gewebeaufbau/-regeneration erreicht wird. Die Stoffwechselaktivität der Zellen, wiederum gemessen mittels des Alamar-Blau-Assays als Referenzparameter für die Zellproliferation und Aktivität der Zellen, ist nach einem Zeitraum von 4 Wochen mit der erfindungsgemäß verwendeten SiX₄-Faser im Vergleich zu herkömmlichen Biomaterialien wie PGA und Kollagen deutlich überlegen: Das Verhältnis für Kollagen : PGA : SiX₄ beträgt 1 : 1,5 : 12 (Fig. 6).

Der oben erwähnte Alamar-Blau-Assay (alamarBlue^{™}-Reduktion) soll hier kurz erläutert werden.
Das Innere von proliferierenden Zellen ist stärker reduziert als das von nicht proliferierenden Zellen. Insbesondere werden die Verhältnisse NADPH/NADP, FADH/FAD, FMNH/FMN, und NADH/NAD während der Proliferation größer. Substanzen wie alamarBlue^{™}, die von diesen Stoffwechsel-Intermediaten reduziert werden, können verwendet werden, um die Proliferation von Zellen zu messen und aufzuzeichnen. Das Redox-Potential von alamarBlue^{™} liegt bei +380 mV (pH 7.0, 25°C). alamarBlue^{™} wird daher reduziert von NADPH (Eo=-320 mV), FADH (Eo=-220 mV), FMNH (Eo=-210mV), NADH (Eo=-320 mV) und Cytochromen (Eo=290 mV bis +80 mV). Da alamarBlue^{™} folglich Elektronen von diesen Substanzen aufnhmen kann, verändert es mit seinem Redoxzustand auch seine Farbe: vom oxidierten, Indigoblauen und nicht-fluoreszierenden Zustand zum reduzierten fluoreszierenden Rosa-Zustand. Das Ausmaß der Proliferation kann dann spektrophotometrisch verfolgt werden, entweder mittels Farbmessung oder mittels Fluoreszenz (siehe hierzu auch die Internetseite der Fa. Biosource Inc., Camarillo, Kalifornien (US), unter der Adresse <www.lucernachem.ch/downloads/biosource/alamarbluebooklet.pdf>).

Die Herstellung des Vlieses erfolgt vorzugsweise, wie oben schon erwähnt, in einer gesättigten alkoholischen Lösung. Das Vlies ist daher steril. Die Vliesgröße ist vollständig frei wählbar und kann an die Größen der gängigen Wundauflagen angepasst werden, typischerweise 10 x 10 cm, 5 x 5 cm oder 2,5 x 2,5 cm (alle andere Ausmaße sind gleichfalls wählbar).

Für die geeignete Lagerung des Vlieses bieten sich zwei unterschiedliche Varianten an: entweder wird das Vlies steril und dicht verpackt (z.B. in Aluminium) und zur späteren Verarbeitung gelagert bzw. verschickt. In die sterile Verpackung kann zusätzlich ein mit Alkohol getränktes Depot, z.B. Watte, eingebracht werden, um die gesättigte Alkohol-Atmosphäre aufrecht zu erhalten. Alternativ dazu wird das Vlies direkt zu einem Multilagen-Verband (Aufbau eines solchen wird nachfolgend und in Fig. 1 exemplarisch beschrieben) weiterverarbeitet, also mit einer wasserdichten oder semipermeablen klebfähigen Membran (z.B. einer Polyurethan- oder Polyesterfolie) verbunden. Nachfolgend wird diese Membran als Klebepflaster 3 oder Membran 3 bezeichnet, auch wenn die Membran/Folie an sich gar nicht klebefähig sein muss.

Die Fasern und Vliese werden während des gesamten Vorbereitungs- und Herstellungszeitraums (von der Herstellung der Fasern bis zum Einbringen des Vlieses in die Wunde) vorzugsweise in einer gesättigten Alkohol-Atmosphäre gehalten, um einer weitergehenden Kondensation des Sihaltigen Fasermaterials und damit verbunden einem Verlust der Biodegradierbarkeit der Faser vorzubeugen. Dies wird z.B. mittels eines sog. In-Line-Herstellungsprozesses realisiert, bei dem bis zum Endprodukt in einer gesättigten Alkohol-Atmosphäre gearbeitet wird. Dies hat außerdem den Vorteil, dass nach erfolgter Herstellung des Vlieses bzw. des Multilagen-Verbandes ein Sterilisationsverfahren (z.B. eine Gamma-Sterilisation) nicht nötig ist.

Ein typischer Multilagen-Verband gemäß der vorliegenden Erfindung, eingepaßt in eine Wunde, ist in Figur 1 dargestellt. Erfindungsgemäß sind aber auch andere Multilagen-Verbände vorstellbar und werden nachfolgend vorgestellt.
Auf das Vlies 1, also zwischen Vlies 1 und Membran/Klebepflaster 3, das gemäß der hier beschriebenen Ausführungsform eine klebefähige Membran/Folie aufweist (das gemäß anderer Ausführungsformen aber auch lediglich eine wasserdichte bzw. semipermeable Membran/Folie sein kann) und in dieser Ausführungsform das Verbandmittel darstellt, wird eine sog. weitere Membran 2 aufgebracht, damit beim Abziehen oder beim Wechsel des oben aufliegenden Klebepflasters 3/der Membran 3 das Vlies 1 nicht aus der Wunde 4 entfernt wird. Das Klebepflaster 3/Die Membran 3 stellt sicher, dass durch die Verwendung des erfindungsgemäßen Multilagen-Verbandes eine sichere Versiegelung der Wunde gegenüber der Außen-Umgebung gegeben ist. Die weitere Membran 2 ist gemäß einer Ausführungsform weder mit dem Vlies 1 noch mit der Membran 3/dem Klebepflaster 3 fest verbunden. Dennoch kann mit der Membran 3/dem Klebepflaster 3 eine feste Verbindung bestehen. Entscheidend ist hierbei, dass beim Entfernen der Membran 3/des Klebepflasters 3 keine Anteile des Vlieses 1 und des neugebildeten Gewebes entfernt werden. Die Anbindung der Membran 2, die aus einem wasserlöslichen Polymer (jedes nicht mit dem Vlies verklebende Polymer) und vorzugsweise aus Carboxymethyl-Cellulose (CMC) besteht, an das Vlies 1 erfolgt z.B. über Wasserstoffbrücken. Die Wahl des Polymers ist dabei nicht entscheidend (auch wasserlösliche Kollagene oder Fibringele können hier eingesetzt werden), da die Membran 2 lediglich dafür sorgt, dass die klebefähige Membran/Folie des Klebepflasters 3 nicht mit dem Vlies 1 verklebt. Diese Ausführungsform ist in Fig. 1 dargestellt.

In einer weiteren Ausführungsform ist die weitere Membran 2 insofern entbehrlich, als das zu verwendende Verbandmittel nicht mit der Wunde verklebt und als weitere Membran 2 fungiert bzw. diese überflüssig macht. Zu derartigen Verbandmitteln, die nicht mit der Wunde verkleben, zählen Alginate (in Kompressenform oder als Tamponade), Kollagenschwämme, PolyurethanSchäume und -Schaumauflagen, Hydrokolloide, Hydrogele und Hydropolymere. In diesem Fall wird die Membran 3 zur Abdichtung der Wunde wiederum entsprechend der Figur 1 mit einem Kleber (auf der die Wunde umgebenden, unbeschädigten Haut) befestigt, wobei der Kleber bevorzugterweise ein Polyacrylat-, ein Kautschuk- oder ein mittels des Heißschmelz-Verfahrens hergestellter Kunstkautschuk-Kleber ist..

Nach einer weiteren Ausführungsform der vorliegenden Erfindung wird auf das Vlies 1 ein Verbandmittel aufgebracht, das einerseits nicht mit der Wunde verklebt (und daher wieder als weitere Membran 2 fungiert bzw. diese überflüssig macht) und das andererseits klebende Eigenschaften besitzt und dadurch die Wunde nach außen abdichtet. Ein solches Verbandmittel kann z.B. aus der Gruppe der 'Foamverbände' (Hydropolymer-Verbände, insbesondere Polyurethan-Foamverbände z.B. Foamverbände von 3M, Silastic von Dow Coming, vertrieben durch Calmic Medical Division, Allevyn von Smith and Nephew, Lyofoam von Seton Healthcare Group plc) ausgewählt sein, denn diese Foamverbände haben u.a. eine starke Flüssigkeitsspeicher-Kapazität (siehe Bello (2000) JAMA 283(6): 716-8; Degreef (1998) Dermatologic Clinics 16(2): 365-75; Findlay (1996) Am Fam Physician 54(5): 1519-28; Habif (1996) Clinical Derm. Mosby, 810-13; Knapp (1999) Ped Clin N Am 46(6):1201-13; Krasner (1995) Prevention Management Pressure Ulcers; Lewis (1996) Med-Surg Nursing, Mosby, p. 199-200; Lueckenotte (1996) Gerontologic Nurs., Mosby, 800-7; PUGP (1995) Am Fam Physician 51(5):1207-22; PUGP (1994) Pressure Ulcer Treatment, AHCPR 95-0653; Way (1991) Current Surgical, Lange, 95-108; oder unter folgenden Internetadressen: http://med2med.de/pages/produktdetail.cfm?prod=85696&katid=6618 und http://wound.smith-nephew.com/de/node.asp?NodeId=2692). Gemäß einer weiteren Ausführungsform der Erfindung kann auf die weitere Membran 2 aber auch vollständig (ersatzlos) verzichtet werden, wenn das Klebepflaster 3 (das in diesem Fall nicht unbedingt eine Klebefähigkeit besitzt) direkt auf das Vlies 1 aufgebracht werden kann, weil sichergestellt ist, dass die klebefähige Membran/Folie des Klebepflasters 3 ausschließlich mit der die Wunde umgebenden Haut eine Klebeverbindung eingeht und somit ein Verkleben des Klebepflasters 3 mit dem Vlies 1 nicht möglich ist. Dies kann z.B. dadurch erreicht werden, dass entweder vor dem Abdeckungsprozess ausschließlich die die Wunde umgebende Haut mit einem Kleber (z.B mit Leukospray^{®} von Baiersdorf) benetzt wird (in diesem Fall besitzt das Klebepflaster 3 selbst also keine Klebefähigkeit und wird daher zutreffender als Membran 3 bezeichnet) oder das Klebepflaster 3 entsprechend der Wundgröße ausgewählt oder zugeschnitten wird (in diesem Fall besitzt das Klebepflaster 3 Klebefähigkeit nur an den Stellen, die nicht mit der Wunde in Berührung gebracht werden sollen).
Das Klebepflaster 3/Die Membran 3 besteht erfindungsgemäß aus einer wasserdichten Folie aus mindestens einem in Wasser unlöslichen Polymer, vorzugsweise PP, PVC oder PU. Es weist darüber hinaus ggf. (siehe die verschiedenen oben geschilderten Ausführungsformen) einen üblicherweise in der Verbandtechnik verwendeten Kleber auf (vorzugsweise ein Polyacrylat- oder ein mittels des Heißschmelz-Verfahrens hergestellter Kunstkautschuk-Kleber, ggf. auch ein Kautschuk-Kleber), der vorzugsweise eine besonders gute Hautverträglichkeit haben sollte. Der Kleber kann bereits bei oder vor der Herstellung des Multilagen-Verbands auf die wasserdichte Folie aufgebracht werden. Er kann aber auch, wie vorstehend erwähnt, erst vom Anwender auf die die Wunde umgebenden Bereiche, oder auf die Membran/Folie, aufgetragen bzw. -gesprüht werden.

Das wasserdichte Klebepflaster 3/Die wasserdichte Membran 3 gewährleistet, dass keine Feuchtigkeit nach außen verdunstet und somit permanent ein feuchtes Wundmilieu aufrechterhalten wird, was zur Resorption der Fasern des Vlieses beiträgt. Die Resorption der Fasern bewirkt auch die Freisetzung von den ggf. an die Fasern gebundenen Substanzen, also z.B. eine Freisetzung und Akkumulation von Ionen (z.B. Ag-Ionen), Medikamenten (z.B. Antibiotika, Corticoide) oder morphogenen Faktoren. Zu diesen morphogenen Faktoren (auch Morphogene genannt), die auch vom Organismus während der Wundheilung gebildet werden, die Wundheilung günstig beeinflussen und für eine gute Wundheilung unerlässlich sind, zählen Interleukine, bone morphogenetic proteins (BMPs), Antikörper, TGF-β und IGF.

Die Wasserlöslichkeit des Polymers der weiteren Membran 2 ermöglicht ein leichtes Ablösen dieser Membran (sofern vorhanden) nach einer gewissen Einwirkungszeit (das wässrige Wundsekret löst nach und nach das Vlies von der weiteren Membran 2 ab, so dass es zu keiner Schädigung des Gewebes beim Abheben der Membran 2 kommt). Vorteilhafterweise werden mit Silber dotierte Polymere als weitere Membran 2 eingesetzt, um die Gefahr einer Infektion zu reduzieren.

Bei besonders großen Wunden (>10 cm²) ist ein schwimmender Verband sinnvoll, da dann die Adhäsionskräfte von weiterer Membran 2 zum Vlies 1 unter Umständen zu groß werden. Als Trennmedium sind in diesen Fällen Hydrogele als dünne (< 5 mm) Schwimmschicht möglich.

Erfindungsgemäß bevorzugte Ausführungsformen werden durch folgende Multilagen-Verbände beschrieben.
1. Ein Multilagen-Verband, der ein Vlies 1, das mit der Wunde in Berührung kommen soll, und eine Membran 3, die wasserdicht ist und mindestens ein in Wasser unlösliches Polymer aufweist, umfaßt, wobei die Membran 3 ein Klebepflaster 3 ist und einen klebefähigen Anteil umfaßt, der mit der die Wunde umgebenden Haut verklebt, und wobei zwischen Membran 3 und Vlies 1 eine lockere, leicht zu lösende Haftverbindung oder gar keine Haftverbindung besteht.
2. Ein Multilagen-Verband, der ein Vlies 1, das mit der Wunde in Berührung kommen soll, und eine Membran 3, die wasserdicht ist und mindestens ein in Wasser unlösliches Polymer aufweist, umfaßt, wobei die Membran 3 keinen klebefähigen Anteil umfaßt und mit der die Wunde umgebenden Haut nur verklebt, wenn auf die die Wunde umgebende Haut ein Kleber aufgetragen worden ist, und wobei zwischen Membran 3 und Vlies 1 eine lockere, leicht zu lösende Haftverbindung oder gar keine Haftverbindung besteht.
3. Ein Multilagen-Verband nach Anspruch 1 oder nach Ausführungsform 1 oder 2, wobei das mindestens eine in Wasser unlösliche Polymer der Membran 3 PP, PVC oder PU ist.
4. Ein Multilagen-Verband nach Ausführungsform 3, wobei die Membran 3 ein selbstklebendes Hydropolymer ist.
5. Ein Multilagen-Verband nach Anspruch 1 oder nach Ausführungsform 1, 2 oder 3, wobei der Multilagen-Verband weiterhin eine weitere Membran 2 zwischen Membran 3 und Vlies 1 umfaßt, die mindestens ein in Wasser lösliches Polymer aufweist.
6. Ein Multilagen-Verband nach Ausführungsform 5, wobei das mindestens eine in Wasser lösliche Polymer CMC ist.
7. Ein Multilagen-Verband nach Ausführungsform 5 oder 6, wobei zwischen weiterer Membran 2 und Vlies 1 eine lockere, leicht zu lösende Haftverbindung besteht.
8. Ein Multilagen-Verband nach Ausführungsform 5 oder 6, wobei zwischen weiterer Membran 2 und Vlies 1 keine Haftverbindung besteht.
9. Ein Multilagen-Verband nach Ausführungsform 5, 6, 7 oder 8, wobei zwischen weiterer Membran 2 und Membran 3 (i) keine, (ii) eine lockere, leicht zu lösende oder (iii) eine feste, nicht zu lösende Haftverbindung besteht.
10. Ein Multilagen-Verband nach Anspruch 1 oder Ausführungsform 1 bis 4, wobei der Multilagen-Verband weiterhin ein Alginat, einen Kollagenschwamm, eine/n Polyurethan-Schaum oder -Schaumauflage, ein Hydrokolloid, ein Hydrogel oder ein Hydropolymer zwischen Membran 3 und Vlies 1 umfaßt.
11. Ein Multilagen-Verband nach Ausführungsform 10, wobei zwischen dem Alginat, dem Kollagenschwamm, dem/der Polyurethan-Schaum oder -Schaumauflage, dem Hydrokolloid, dem Hydrogel oder dem Hydropolymer und Vlies 1 eine lockere, leicht zu lösende Haftverbindung besteht.
12. Ein Multilagen-Verband nach Ausführungsform 10, wobei zwischen dem Alginat, dem Kollagenschwamm, dem/der Polyurethan-Schaum oder -Schaumauflage, dem Hydrokolloid, dem Hydrogel oder dem Hydropolymer und Vlies 1 keine Haftverbindung besteht.
13. Ein Multilagen-Verband nach Ausführungsform 10, 11 oder 12, wobei zwischen dem Alginat, dem Kollagenschwamm, dem/der Polyurethan-Schaum oder -Schaumauflage, dem Hydrokolloid, dem Hydrogel oder dem Hydropolymer und Membran 3 (i) keine, (ii) eine lockere, leicht zu lösende oder (iii) eine feste, nicht zu lösende Haftverbindung besteht.

Auf den auf dieser und der vorangehenden Seite in seinen verschiedenen Ausführungsformen beschriebenen erfindungsgemäßen Multilagen-Verband läßt sich bei Bedarf noch (weiteres) Verbandmaterial (z.B. Verbandmull) oder sonstiges Material (z.B. zum Polstern oder Schützen der Wunde) aufbringen. Im Rahmen dieser Offenbarung hierin wird also zwischen Verbandmittel (als Bestandteil des Multilagen-Verbands) und Verbandmaterial unterschieden.

Bevor das Vlies auf/in die Wunde gebracht bzw. zum Multilagen-Verband verarbeitet wird (also nach seiner Herstellung, also während seiner Lagerung bzw. seinem Transport, sei es als reiner Vlies oder als Multilagen-Verband), wird es vorzugsweise an der Oberfläche, die später mit der Wunde in Berührung kommt, durch eine dichte Membran isoliert, die ein Entweichen des Alkohols verhindert. Die Membran wird vom Anwender ggf. unmittelbar vor dem Aufbringen in die Wunde abgezogen. Bei dieser bevorzugten Ausführungsform muss allerdings bedacht werden, dass Alkohol, wie oben erläutert, zwar die Sterilität und die Faserchemie stabilisiert, jedoch äußerst schmerzhaft ist, wenn er mit der Wunde in Berührung gebracht wird. Deshalb kann gemäß einer weiteren bevorzugten Ausführungsform physiologische Kochsalz-Lösung als Medium verwendet werden. Alternativ kann der Alkohol vor der Anwendung verdunstet oder ausgewaschen werden, was jedoch die Anwendung insgesamt kompliziert.

Typische Verbandmittel, das mit dem Vlies ausgerüstet werden kann, sind z.B. die folgenden Produkte (Marken-Bezeichnungen):
Dermaplast Film/Active und Hydractive®; Hydrofilm Plus®; Hydrocoll® (Hartmann); Comfeel (-Plus)®, Biatain®, Seasorb®, Contreet® (Coloplast)
Cutinova Hydro®, Acticoat®, Allevyn® (Smith&Nephew)

Das Vlies kann aber auch mit allen Variationen bereits am Markt befindlicher Produkte kombiniert werden, z.B. mit folgenden Produkten der Firma Smith & Nephew (allesamt eingetragene Marken):
Hydrogel-Verbände, IntraSite Conformable, IntraSite Gel, hydroselektive Wundauflagen, Cutinova Hydro (z.B. hydrozelluläre Schaumstoffwundauflagen), Allevyn Produktgruppe (z.B. Alginate, antimikrobielle Wundauflagen, enzymatisches Debridement, geruchsabsorbierende Verbände, Post-Operativ-Verbände), Cutiplast Steril, Hansapor Steril, OpSite Post-Op, Primapore (z.B. Spezialverbände), Allevyn Tracheostomy, Cavi-Care, EXU-DRY.

So kann das Vlies z.B. mit Polyurethan- oder PVA-Schwammverbänden kombiniert werden, um die Saugkraft bei stark sezemierenden Wunden zu erhöhen. Die genannten Vorteile der Erfindung sind insbesondere bei der Anwendung von Vakuumsystemen (z.B. V.A.C®) ersichtlich, z.B. bei der Behandlung septischer Wunden und der Notwendigkeit von Antibiotika-Spülungen. Darüber hinaus kann das Vlies auch mit den weiter oben schon erwähnten Alginat-Tamponaden als Abdeckung im Rahmen hydrokolloider Wundverbände kombiniert werden.

Die Form des Multilagen-Verbandes kann entsprechend der Wundlokalisation bzw. ihrer Form und Ausmaße variiert werden, um eine möglichst exakte Anpassung an die Wundanatomie zu erreichen. Eine mögliche Form des Verbandes ist die Schmetterlingsform, die im Analbereich Anwendung finden kann.

## Patentansprüche

1. Multilagen-Verband, der wenigstens den folgenden Aufbau hat:
ein Vlies 1, das mit der Wunde in Berührung kommen soll, und
eine Membran 3, die wasserdicht ist und mindestens ein in Wasser unlösliches Polymer aufweist, wobei die Membran 3 entweder ein Klebepflaster 3 ist und einen klebefähigen Anteil umfaßt, der mit der die Wunde umgebenden Haut verklebt, oder wobei die Membran keinen klebefähigen Anteil umfaßt und mit der die Wunde umgebenden Haut nur verklebt, wenn auf die die Wunde umgebende Haut ein Kleber aufgetragen worden ist, wobei das Vlies 1 biologisch degradierbare und/oder resorbierbare Faserstrukturen aufweist, die sich erhalten lassen, indem aus einer Spinnmasse Fäden gezogen werden, wobei die Spinnmasse eine oder mehrere partiell oder vollständig hydrolytisch kondensierte Verbindungen des Siliciums enthält, die sich durch hydrolytische Kondensation ableiten von Monomeren der allgemeinen Formel SiX4, in der die Reste X gleich oder verschieden sind und Hydroxy, Wasserstoff, Halogen, Amino, Alkoxy, Alkyloxy, Alkylcarbonyl oder Alkoxycarbonyl bedeuten oder sich von Alkyl-Resten ableiten und durch Sauerstoff- oder Schwefel-Atome oder durch Amino-Gruppen unterbrochen sein können,
und wobei zwischen Membran 3 und Vlies 1 eine lockere, leicht zu lösende Haftverbindung oder gar keine Haftverbindung besteht.

2. Der Multilagen-Verband nach Anspruch 1, wobei das mindestens eine in Wasser unlösliche Polymer der Membran 3 PP, PVC oder PU ist.

3. Der Multilagen-Verband nach Anspruch 1 oder 2, wobei die Membran 3 ein selbstklebendes Hydropolymer ist.

4. Der Multilagen-Verband nach einem der vorhergehenden Ansprüche, wobei der Multilagen-Verband weiterhin eine weitere Membran 2 zwischen Membran 3 und Vlies 1 umfaßt, die mindestens ein in Wasser lösliches Polymer aufweist.

5. Der Multilagen-Verband nach Anspruch 4, wobei das mindestens eine in Wasser lösliche Polymer CMC ist.

6. Der Multilagen-Verband nach Anspruch 4 oder 5, wobei zwischen weiterer Membran 2 und Vlies 1 eine lockere, leicht zu lösende Haftverbindung besteht.

7. Der Multilagen-Verband nach Anspruch 4 oder 5, wobei zwischen weiterer Membran 2 und Vlies 1 keine Haftverbindung besteht.

8. Der Multilagen-Verband nach einem der Ansprüche 4 bis 7, wobei zwischen weiterer Membran 2 und Membran 3 (i) keine, (ii) eine lockere, leicht zu lösende oder (iii) eine feste, nicht zu lösende Haftverbindung besteht.

9. Der Multilagen-Verband nach einem der Ansprüche 1 bis 3, wobei der Multilagen-Verband weiterhin ein Alginat, einen Kollagenschwamm, eine/n Polyurethan-Schaum oder - Schaumauflage, ein Hydrokolloid, ein Hydrogel oder ein Hydropolymer zwischen Membran 3 und Vlies 1 umfaßt.

10. Der Multilagen-Verband nach Anspruch 9, wobei zwischen dem Alginat, dem Kollagenschwamm, dem/der Polyurethan-Schaum oder -Schaumauflage, dem Hydrokolloid, dem Hydrogel oder dem Hydropolymer und Vlies 1 eine lockere, leicht zu lösende Haftverbindung besteht.

11. Der Multilagen-Verband nach Anspruch 9, wobei zwischen dem Alginat, dem Kollagenschwamm, dem/der Polyurethan-Schaum oder -Schaumauflage, dem Hydrokolloid, dem Hydrogel oder dem Hydropolymer und Vlies 1 keine Haftverbindung besteht.

12. Der Multilagen-Verband nach einem der Ansprüche 9 bis 11, wobei zwischen dem Alginat, dem Kollagenschwamm, dem/der Polyurethan-Schaum oder -Schaumauflage, dem Hydrokolloid, dem Hydrogel oder dem Hydropolymer und Membran 3 (i) keine, (ii) eine lockere, leicht zu lösende oder (iii) eine feste, nicht zu lösende Haftverbindung besteht.

13. Der Multilagen-Verband nach einem der vorherigen Ansprüche, wobei die Reste X gleich sind und Ethyl bedeuten.

14. Der Multilagen-Verband nach einem der vorherigen Ansprüche, wobei die hydrolytische Kondensation in Gegenwart von einer (oder mehreren) Aminosäuren und/oder von einem (oder mehreren) Peptiden und/oder von einem (oder mehreren) DNA-Molekül(en) bzw. - Fragmenten erfolgt.

## Claims

1. Multilayer dressing which has at least the following structure:
a nonwoven 1, which is intended to come into contact with the wound, and a membrane 3, which is water-impermeable and includes at least one water-insoluble polymer, where the membrane 3 is either an adhesive plaster 3 and includes an adhesive portion which adheres to the skin surrounding the wound, or where the membrane includes no adhesive portion and adheres to the skin surrounding the wound only when an adhesive has been applied to the skin surrounding the wound, where the nonwoven 1 includes biodegradable and/or - absorbable fibrous structures which can be obtained by drawing threads from a spinning dope, where the spinning dope comprises one or more partially or completely hydrolytically condensed compounds of silicon which are derived by hydrolytic condensation from monomers of the general formula SiX₄ in which the X radicals are identical or different and are hydroxy, hydrogen, halogen, amino, alkoxy, alkyloxy, alkylcarbonyl or alkoxycarbonyl or are derived from alkyl radicals and may be interrupted by oxygen or sulfur atoms or by amino groups, and where the bonding between membrane 3 and nonwoven 1 is loose and easily broken or non-existent.

2. The multilayer dressing according to Claim 1, where the at least one water-insoluble polymer of the membrane 3 is PP, PVC or PU.

3. The multilayer dressing according to Claim 1 or 2, where the membrane 3 is a self-adhesive hydropolymer.

4. The multilayer dressing according to any of the preceding claims, where the multilayer dressing further comprises a further membrane 2 between membrane 3 and nonwoven 1, which includes at least one water-soluble polymer.

5. The multilayer dressing according to Claim 4, where the at least one water-soluble polymer is CMC.

6. The multilayer dressing according to Claim 4 or 5, where the bonding between further membrane 2 and nonwoven 1 is loose and easily broken.

7. The multilayer dressing according to Claim 4 or 5, where the bonding between further membrane 2 and nonwoven 1 is non-existent.

8. The multilayer dressing according to any of Claims 4 to 7, where the bonding between further membrane 2 and membrane 3 (i) is non-existent, (ii) is loose and easily broken or (iii) is stable and unbreakable.

9. The multilayer dressing according to any of Claims 1 to 3, where the multilayer dressing further comprises an alginate, a collagen sponge, a polyurethane foam or foam layer, a hydrocolloid, a hydrogel or a hydropolymer between membrane 3 and nonwoven 1.

10. The multilayer dressing according to Claim 9, where the bonding between the alginate, the collagen sponge, the polyurethane foam or foam layer, the hydrocolloid, the hydrogel or the hydropolymer and nonwoven 1 is loose and easily broken.

11. The multilayer dressing according to Claim 9, where the bonding between the alginate, the collagen sponge, the polyurethane foam or foam layer, the hydrocolloid, the hydrogel or the hydropolymer and nonwoven 1 is non-existent.

12. The multilayer dressing according to any of Claims 9 to 11, where the bonding between the alginate, the collagen sponge, the polyurethane foam or foam layer, the hydrocolloid, the hydrogel or the hydropolymer and membrane 3 (i) is non-existent, (ii) is loose and easily broken or (iii) is stable and unbreakable.

13. The multilayer dressing according to any of the preceding claims, where the X radicals are identical and are ethyl.

14. The multilayer dressing according to any of the preceding claims, where the hydrolytic condensation takes place in the presence of one (or a plurality of) amino acids and/or of one (or a plurality of) peptides and/or of one (or a plurality of) DNA molecule(s) or fragments.

## Revendications

1. Pansement multicouche, qui comprend au moins la structure suivante:
- un non tissé 1, qui doit venir en contact avec la plaie, et
- une membrane 3, qui est étanche à l'eau et qui comprend au moins un polymère insoluble dans l'eau, dans lequel la membrane 3 est un pansement adhésif 3 et comprend une partie qui adhère à la peau entourant la plaie, ou dans lequel la membrane ne comporte pas de partie adhésive et n'adhère à la peau entourant la plaie que si un adhésif a été appliqué sur la peau entourant la plaie, dans lequel le non tissé 1 comprend des structures de fibres biodégradables et/ou résorbables, qui peuvent être obtenues en étirant des fibres hors d'une masse de filage, dans lequel la masse de filage contient un ou plusieurs composés du silicium condensés, partiellement ou totalement par hydrolyse, qui sont obtenus par condensation hydrolytique de monomères de la forme générale SiX4, dans laquelle les radicaux X sont identiques ou différents et représentent des radicaux hydroxy, hydrogène, halogène, amino, alkoxy, alkyloxy, alkylcarbonyle ou alkoxycarbonyle ou sont dérivés de radicaux alkyle et peuvent être interrompus par des atomes d'oxygène ou de soufre ou par des groupes aminés, et dans lequel il existe entre la membrane 3 et le non tissé 1 une liaison par adhérence aisée à détacher ou pas de liaison par adhérence du tout.

2. Pansement multicouche selon la revendication 1, dans lequel l'au moins un polymère insoluble dans l'eau de la membrane 3 est le PP, le PVC ou le PU.

3. Pansement multicouche selon la revendication 1 ou la revendication 2, dans lequel la membrane 3 est un hydropolymère autoadhésif.

4. Pansement multicouche selon l'une quelconque des revendications précédentes, dans lequel le pansement multicouche comprend en outre une autre membrane 2, entre la membrane 3 et le non tissé 1, qui présente au moins un polymère soluble dans l'eau.

5. Pansement multicouche selon la revendication 4, dans lequel l'au moins un polymère soluble dans l'eau est le CMC.

6. Pansement multicouche selon la revendication 4 ou la revendication 5, dans lequel il existe une liaison par adhérence légère, aisée à détacher, entre l'autre membrane 2 et le non tissé 1.

7. Pansement multicouche selon la revendication 4 ou la revendication 5, dans lequel il n'existe pas de liaison par adhérence entre l'autre membrane 2 et le non tissé 1.

8. Pansement multicouche selon l'une quelconque des revendications 4 à 7, dans lequel il existe entre l'autre membrane 2 et la membrane 3: (i) aucune liaison par adhérence, (ii) une liaison par adhérence légère, aisée à détacher, ou (iii) une liaison par adhérence solide, impossible à détacher.

9. Pansement multicouche selon l'une quelconque des revendications 1 à 3, dans lequel le pansement multicouche comprend en outre un alginate, une mousse de collagène, une mousse ou une couche de mousse de polyuréthane, un hydrocolloïde, un hydrogel ou un hydropolymère entre la membrane 3 et le non tissé 1.

10. Pansement multicouche selon la revendication 9, dans lequel il existe une liaison par adhérence légère, aisée à détacher, entre l'alginate, la mousse de collagène, la mousse/la couche de mousse de polyuréthane, l'hydrocolloïde, l'hydrogel ou l'hydropolymère et le non tissé 1.

11. Pansement multicouche selon la revendication 9, dans lequel il n'existe pas de liaison par adhérence entre l'alginate, la mousse de collagène, la mousse/la couche de mousse de polyuréthane, l'hydrocolloïde, l'hydrogel ou l'hydropolymère et le non tissé 1.

12. Pansement multicouche selon l'une quelconque des revendications 9 à 11, dans lequel il existe, entre l'alginate, la mousse de collagène, la mousse/la couche de mousse de polyuréthane, l'hydrocolloïde, l'hydrogel ou l'hydropolymère et la membrane 3: (i) aucune liaison par adhérence, (ii) une liaison par adhérence légère, aisée à détacher, ou (iii) une liaison par adhérence solide, impossible à détacher.

13. Pansement multicouche selon l'une quelconque des revendications précédentes, dans lequel les radicaux X sont identiques et représentent l'éthyle.

14. Pansement multicouche selon l'une quelconque des revendications précédentes, dans lequel la condensation hydrolytique est effectuée en présence d'un (ou de plusieurs) acide(s) aminé(s) et/ou d'un (ou de plusieurs) peptide(s) et/ou d'un(e) (ou de plusieurs) molécule(s) ou fragment(s) d'ADN.
